# EUROPÄISCHE PATENTANMELDUNG

(11) **EP 2 883 536 A1**
(43) Veröffentlichungstag der Anmeldung: **17.06.2015**
(21) Anmeldenummer: 14159773.2
(22) Anmeldetag: 14.03.2014
(51) Int. Cl.: A61K 8/37, A61K 8/39, A61Q 19/00, A61K 8/891, A61K 8/92, A61K 8/06

(54) **Herstellung von Emulsionen enthaltend Wachse im Kaltverfahren**

(30) Priorität: 11.12.2013 DE 102013225549
(71) Anmelder: Henkel AG & Co. KGaA, 40589 Düsseldorf (DE)
(72) Erfinder: Keßler-Becker, Daniela, 51371 Leverkusen (DE); Groehn, Susanne, 40229 Düsseldorf (DE); Struwe, Alexandra, 47877 Willich (DE)

(57) **Zusammenfassung**

Die vorliegende Erfindung betrifft Hautkosmetika, insbesondere wässrige Zusammensetzungen enthaltend Öle, Wachse und mindestens einen ausgewählten Emulgator.

## Beschreibung

Die vorliegende Erfindung betrifft Hautkosmetika, insbesondere wässrige Zusammensetzungen enthaltend Öle, Wachse und mindestens einen ausgewählten Emulgator.

Kosmetika mit Ölen und Wachsen sind dem Fachmann bekannt. Diese werden beispielsweise in der Hautkosmetik als UV-Schutzkosmetikum, als Antitranspirants, als Creme, Lotion oder zur dekorativen Kosmetik als Schminke oder Lippenstiften eingesetzt.

Problematisch bei der Formulierung von Kosmetika mit Ölen und Wachsen ist neben der Stabilität die Herstellung, welche üblicherweise nur in Heißverfahren möglich ist.

Bei Kosmetika mit hohem Wachs- und Ölgehalt wird oft ein unangenehmes schmieriges Gefühl wahrgenommen. Der Verbraucher spürt jeweils die Beschichtung mit dem Kosmetikum nachhaltig. Er wünscht sich auf der Haut ein samtiges, leichtes Hautgefühl und eine gute sowie gleichmäßige Verteilbarkeit. Die Herstellung von Kosmetika enthaltend Wachse und Öle erfolgt üblicherweise so, dass die Wachse aufgeschmolzen werden und dann erst mit Emulgatoren, Wasser und anderen Inhaltsstoffen verarbeitet werden. Dieses Verfahren ist sehr energieaufwendig.

Das Datenblatt des Handelsproduktes Dracorin® GOC beschreibt den Emulgator Dracorin® GOC als einen Emulgator um damit Emulsionen in einem kalten, heiß/kalten oder heißen Emulgierverfahren Emulsionen herzustellen. Im Datenblatt sind keine Beispiel für einen kalt/kalt Prozess mit festen Wachsen enthalten. Alle Beispiele gemäß dem kalt/kalt Emulgierverfahren enthalten ausschließlich Öle.

Aufgabe der vorliegenden Anmeldung war es daher, ein lagerstabiles Kosmetikum, bereitzustellen, welches Öle und Wachse enthält und sich leicht auf einem Substrat, insbesondere der Haut, verteilen lässt. Ferner soll das besagte Kosmetikum ein samtiges und leichtes Hautgefühl vermitteln. Gleichzeitig soll die Herstellung mit einem geringeren Energieaufwand und daher ausschließlich in einem alle Prozessschritte umfassenden Kaltprozess erfolgen.

Es wurde gefunden, dass die gestellte Aufgabe durch eine wachs- und ölhaltige Zusammensetzung enthaltend mindestens einen ausgewählten Emulgator, sowie Öle und Wachse gelöst wird.

Ein erster Erfindungsgegenstand sind daher kosmetische Mittel, enthaltend in einem geeigneten kosmetischen Träger jeweils bezogen auf das Gesamtgewicht des Mittels
- ein oder mehrere flüssige Öle in einer Gesamtmenge von 0,1 bis 30,0 Gew.-%,
- ein oder mehrere Wachse mit einem Schmelzpunkt von 20 bis 90 °C in einer Gesamtmenge von 0,01 bis 10,0 Gew.-%,
- mindestens einen Emulgator, ausgewählt aus den Emulgatoren mit den INCI - Bezeichnungen "Glyceryl Oleate Citrate, Caprylic/Capric Triglyceride", "Polyglyceryl-4 Laurate/Succinate", "Polyglyceryl-4 Laurate/Sebacate (and) Polyglyceryl-4 Caprylate/Caprate", "Glyceryl Stearate (and) Polyglyceryl-6 Palmitate/ Succinate (and) Cetearyl Alcohol Polyglyceryl-4 Laurate/Sebacate (and) Polyglyceryl-6 Caprylate/Caprate" sowie deren Mischungen in einer Gesamtmenge von 0,03 bis 10,0 Gew.-% und
- gegebenenfalls weitere Inhaltsstoffe,
- und dadurch gekennzeichnet, dass die Herstellung der Mittel ausschließlich in einem Kaltverfahren erfolgt.

Überraschenderweise wurde weiterhin gefunden, dass die kosmetischen Mittel selbst dann kalt hergestellt werden können, wenn partikelförmig dispergierte Feststoffe in den Mitteln enthalten sind.

Alle Angaben über die Aggregatzustände von Stoffen (fest, flüssig, gasförmig) in dieser Anmeldung beziehen sich auf Normalbedingungen. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Unter einem Öl ist erfindungsgemäß ein flüssiger Stoff zu verstehen, der bei Normalbedingungen in bidestilliertem Wasser zu weniger als 1 Gew.-% mischbar ist.

Ein "Kaltprozess" im Sinne der vorliegenden Erfindung ist ein Prozess, bei welchem jeder Verfahrensschritt bei einer Temperatur im Bereich der Raumtemperatur, also zwischen 18 °C und 30 °C, vorzugsweise zwischen 20 °C und 25 °C durchgeführt wird. Insbesondere wird das Wachs nicht wie in Verfahren gemäß dem Stand der Technik mit allen anderen hydrophoben Anteilen und Ölen aufgeschmolzen.

In einer bevorzugten Ausführungsform wird das flüssige Öl ausgewählt aus mindestens einer Verbindung der Gruppe, die gebildet wird aus flüssigen Silikonen; Esterölen; Trifettsäureester von gesättigten und/oder ungesättigten, linearen und/oder verzweigten C₆- bis C₂₂-Fettsäuren mit Glycerin; pflanzlichen Ölen; flüssigen Paraffinölen; Isoparaffinölen; flüssigen synthetischen Kohlenwasserstoffen; flüssigen Di-n-alkylethern; Dicarbonsäureestern; symmetrischen, unsymmetrischen oder cyclischen Estern der Kohlensäure.

Der erfindungsgemäße kosmetische Träger ist bevorzugt wässrig und/oder wässrig-alkoholisch. Unter "wässrig" wird verstanden, dass die erfindungsgemäßen kosmetischen Mittel bevorzugt mindestens 50 Gew.-%, mehr bevorzugt mindestens 55 Gew.-%, besonders bevorzugt mindestens 60 Gew.-% und insbesondere mindestens 65 Gew.-% Wasser enthalten.

Fakultativ können die kosmetischen Mittel zusätzlich maximal 20 Gew.-%, bevorzugt maximal 15 Gew.-%, mehr bevorzugt maximal 10 Gew.-% und insbesondere maximal 7,5 Gew.-% mindestens eines Alkohols enthalten.

Geeignete Alkohole sind Ethanol, 1-Propanol, 2-Propanol, Isopropanol, 1,2-Propylenglycol, 1-Butanol, 2-Butanol, Isobutanol, 1,2-Butylenglycol, 1-Pentanol, 2-Pentanol, 1,2-Pentandiol, 1-Haxanol, 2-Hexanol, 1,2-Hexandiol, Glycerin, Sorbitol sowie aromatische Alkohole wie beispielsweise Phenoxyethanol.

Besonders bevorzugt sind Ethanol, 1,2-Propylenglycol, Sorbitol und Glycerin. Insbesondere bevorzugt sind Ethanol und/oder Glycerin.

Am bevorzugtesten enthält der erfindungsgemäße kosmetische Träger neben mindestens 50 Gew.% Wasser maximal 15 Gew.%, insbesondere maximal 7,5 Gew.% Glycerin.

In einer bevorzugten Ausführungsform der Erfindung weisen die kosmetischen Mittel einen pH-Wert im Bereich von 3,5 bis 7,0 mehr bevorzugt von 4 bis 7,0 und insbesondere von 4,5 bis 7 auf.

Es ist erfindungsgemäß bevorzugt, wenn das erfindungsgemäße Mittel bezogen auf das Gewicht der Zusammensetzung mindestens ein flüssiges Öl in einer Gesamtmenge insbesondere von 0,1 bis 30 Gew.-%, insbesondere von 0,3 bis 20 Gew.-% und ganz besonders von 0,5 bis 15 Gew.% in der erfindungsgemäßen Zusammensetzung enthalten sind.

Als ein bevorzugtes flüssiges Öl enthält die erfindungsgemäße Zusammensetzung mindestens ein Silikonöl.

Es ist erfindungsgemäß bevorzugt, wenn das erfindungsgemäße Mittel bezogen auf das Gewicht der Zusammensetzung Silikonöl in einer Gesamtmenge von 0,1 bis 30 Gew.-%, insbesondere von 0,1 bis 20 Gew.-%, in der erfindungsgemäßen Zusammensetzung enthält.

Wiederum bevorzugt werden die Silikonöle ausgewählt, aus mindestens einer Verbindung aus der Gruppe, die gebildet wird aus:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter (per)fluorierten Gruppen;
(iii) oder deren Gemischen.

Ganz besonders bevorzugte kosmetische Mittel sind dadurch gekennzeichnet, dass sie als Öl mindestens ein Silikon der Formel (Si-1)

(CH₃)₃Si-[O-Si(CH₃)₂]ₓ-Si(CH₃)₃ (Si-1),

enthalten, in der x für eine Zahl von 0 bis 200, vorzugsweise von 0 bis 100, weiter bevorzugt von 0 bis 20, steht.

Die erfindungsgemäß bevorzugten kosmetischen Mittel enthalten als Öl mindestens ein Silikon der vorstehenden Formel (Si-1). Diese Silikone werden nach der INCl-Nomenklatur als Dimethicone bezeichnet.

Erfindungsgemäß ebenfalls bevorzugte Silikonöle sind ausgewählt aus Silikonen der Formel (Si-2) wobei x ausgewählt ist aus ganzen Zahlen von 1 - 20, bevorzugt 1 - 3.

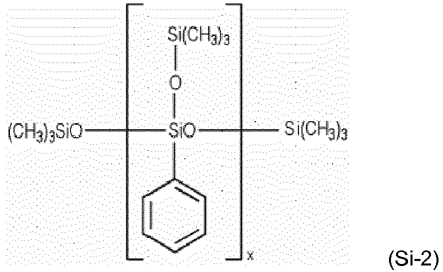

Ein bevorzugtes Silikonöl der Formel (Si-2) ist erhältlich unter der INCl-Bezeichnung Phenyl Trimethicone.

Selbstverständlich können auch Mischungen der o.g. Silikone der Formel (Si-1) und (Si-2) als Silikonöl in den bevorzugten erfindungsgemäßen Mitteln enthalten sein.

Bevorzugte erfindungsgemäß einsetzbare Silikonöle, insbesondere gemäß Formel (Si-1), weisen bei 25°C eine kinematische Viskosität von 1 bis 200 mm²s⁻¹, besonders bevorzugt von 5 bis 100 mm²s⁻¹. Solche Silikonöle sind beispielsweise als Dimethicone unter dem Handelsnamen Xiameter PMX 200 Sil Fluid 50 CS (früher: Dow Corning 200 fluid 50 cSt) im Handel erhältlich.

Im Rahmen einer besonders bevorzugten Ausführungsform der Erfindung sind solche erfindungsgemäßen kosmetischen Mittel bevorzugt, die als flüssiges Öl mindestens einen Ester der Formel (I) enthalten 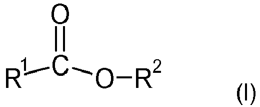 worin R¹ und R² unabhängig voneinander für eine lineare oder verzweigte Kohlenwasserstoffgruppe mit 3 bis 22 Kohlenstoffatomen stehen.

Es ist besonders bevorzugt, wenn gemäß Formel (I) R¹ eine lineare oder verzweigte Kohlenwasserstoffgruppe mit 3 bis 22 Kohlenstoffatomen und R² eine verzweigte Kohlenwasserstoffgruppe mit 3 bis 22 Kohlenstoffatomen ist.

Bevorzugte flüssige Öle werden ausgewählt aus Estern gemäß Formel (I) von C₆ bis C₂₂ - Fettsäuren (R¹ = linearer oder verzweigter C₅-C₂₁ Kohlenwasserstoff) mit C₃ bis C₂₂ - Fettalkoholen (R² = linearer oder verzweigter C₃ bis C₂₂ Kohlenwasserstoff).

Bevorzugte Beispiele für eingesetzte Fettsäurenanteile in den Estern der Formel (I) sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren Mischungen, wie z.B. die technischen Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Bevorzugte Beispiele für die Fettalkoholanteile in den Estern der Formel (I) sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren Mischungen, wie z.B. die technischen Mischungen, die bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen.

Erfindungsgemäß besonders bevorzugt, wird die Verbindung gemäß Formel (I) ausgewählt unter 2-Ethylhexylpalmitat, 2-Ethylhexylstearat, 2-Ethylhexylcocoat, 2-Ethylhexyllaurat, 2-Ethylhexylisostearat, Hexyldecylstearat, Hexyldecyllaurat, Isodecylneopentanoat, Isononylisononanoat, Isopropylmyristat, Isopropylpalmitat, Isopropylstearat, Isopropylisostearat, Isopropyloleat, Isooctylstearat, Isononansäure-C₁₆₋₁₈-alkylester, Isononylstearat, Isocetylstearat, Isononylisononanoat, Isotridecylisononanoat, Cetearylisononanoat, 2-Octyldodecylpalmitat, 2-Octyldodecylmyristat, 2-Octyldodecyllaurat, 2-Octyldodecylstearat, Butyloctansäure-2-butyloctanoat, Kokosfettalkoholcaprinat/-caprylat, n-Butylstearat, n-Hexyllaurat, n-Decyloleat, Cetyloleat, Oleyloleat, Oleylerucat, Erucyloleat, Erucylerucat, Gemischen aus zwei oder mehreren der vorgenannten Verbindungen.

Es ist erfindungsgemäß besonders bevorzugt, die jeweiligen Kohlenwasserstoffreste der Ester der Formel (I), insbesondere im Rahmen der bevorzugten Ausführungsformen besagter Ester (*vide supra*), aus gesättigten Kohlenwasserstoffen auszuwählen.

Die erfindungsgemäßen Mittel enthalten die Verbindungen der Formel (I) bevorzugt in einer Menge von 0,1 bis 30 Gew.-%, insbesondere von 0,3 bis 20 Gew.-%, jeweils bezogen auf das Gesamtgewicht des Mittels.

Im Rahmen dieser Ausführungsform der Erfindung sind wiederum solche erfindungsgemäßen kosmetischen Mittel bevorzugt, die als flüssiges Öl zumindest enthalten
- mindestens einen Ester der Formel (I) 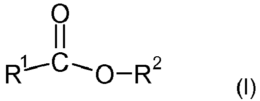 worin R¹ und R² unabhängig voneinander für eine lineare oder verzweigte Kohlenwasserstoffgruppe mit 3 bis 20 Kohlenstoffatomen stehen, und
- mindestens ein Silikonöl.

Im Rahmen dieser Kombination sind wiederum die bevorzugten Ausführungsformen der Verbindungen der Formel (I) und der Silikonöle als besonders bevorzugte Komponenten einsetzbar.

Im Rahmen obiger Ausführungsform der Erfindung sind solche erfindungsgemäßen kosmetischen Mittel besonders bevorzugt, die als flüssiges Öl zumindest enthalten,
- einen oder mehrere Ester der Formel (I) 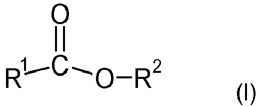 worin R¹ und R² unabhängig voneinander für eine lineare oder verzweigte Kohlenwasserstoffgruppe mit 3 bis 20 Kohlenstoffatomen stehen, in einer Gesamtmenge von 1 bis 20, insbesondere von 5 bis 15 Gew.-%,
- Silikonöl in einer Gesamtmenge von 0,1 bis 30 Gew.-%, insbesondere von 0,1 bis 20 Gew.-%,

Im Rahmen dieser Kombination sind wiederum die bevorzugten Ausführungsformen der Verbindungen der Formel (I) und der Silikonöle als besonders bevorzugte Komponenten einsetzbar.

Als flüssiges Öl erfindungsgemäß geeignet sind auch Triglyceridöle wie die flüssigen Anteile des Rindertalgs sowie synthetische Triglyceridöle. Bevorzugte Triglyceridöle werden ausgewählt unter den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Rapsöl, Olivenöl, Sesamöl, Distelöl, Weizenkeimöl, Pfirsichkernöl und die flüssigen Anteile des Kokosöls, Glyceryltriölsäureester (Triolein), Capric/Caprylic Triglyceride, Glyceryltrüsostearin, Glyceryltriisopalmitat und Mischungen aus zweien oder mehreren der vorgenannten Verbindungen.

Bevorzugte pflanzliche Öle werden ausgewählt aus mindestens einem Vertreter der Gruppe, die gebildet wird aus Amaranthöl, Sonnenblumenöl, Olivenöl, Sojaöl, Rapsöl, Mandelöl, Jojobaöl, Orangenöl, Aprikosenkernöl, Macadamianussöl, Weizenkeimöl, Pfirsichkernöl und die flüssigen Anteile des Kokosöls.

Bevorzugte Di-n-alkylether werden ausgewählt aus Di-n-alkylethern mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.

Bevorzugte Dicarbonsäureester werden ausgewählt aus mindestens einer Verbindung der Gruppe, die gebildet wird, aus Di-n-butyladipat, Di-(2-ethylhexyl)-adipat, Di-(2-ethylhexyl)-succinat und Diisotridecylacelaat sowie Diolestern wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat.

Natürliche und synthetische Kohlenwasserstoffe, wie beispielsweise Paraffinöle, C₁₈-C₃₀-Isoparaf-fine, insbesondere Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (erhältlich z. B. unter dem Handelsnamen Cetiol^{®}S von Cognis) gehören ebenfalls zu den erfindungsgemäß bevorzugt einsetzbaren flüssigen Ölen.

Weitere erfindungsgemäß bevorzugte flüssige Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.

Weitere erfindungsgemäß bevorzugte flüssige Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/ Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Weitere erfindungsgemäß bevorzugte flüssige Öle sind ausgewählt aus den symmetrischen, unsymmetrischen oder cyclischen Estern der Kohlensäure mit Alkanolen, z. B. Glycerincarbonat, Dicaprylylcarbonat (Cetiol^{®} CC) oder die Ester gemäß der Lehre der DE 19756454 A1.

Weiterhin enthalten die erfindungsgemäßen Mittel zwingend mindestens ein Wachs. Unter Wachsen werden im Allgemeinen Feststoffe verstanden, die Rekationsprodukte von Carbonsäuren und Alkoholen sind. Diese Reaktionsprodukte sind als Wachs fest bis brüchig hart, bis 20°C knetbar und schmelzen bei 20°C bis 90°C.

Erfindungsgemäße Wachse weisen bevorzugt einen Schmelzpunkt in einem Bereich von 20°C bis 90°C, besonders bevorzugt in einem Bereich von 30°C bis 60°C, ganz besonders bevorzugt in einem Bereich von 30°C bis 40°C, auf.

Es ist erfindungsgemäß bevorzugt, wenn mindestens die Hälfte der Menge des enthaltenen Wachses (besonders bevorzugt mindestens 75% der Menge des enthaltenen Wachses) im flüssigen Öl des erfindungsgemäßen kosmetischen Mittels gelöst vorliegt. Wenn im Zusammenhang mit dieser Ausführungsform mindestens ein Ester der Formel (I) (*vide supra*) in der flüssigen Ölkomponente enthalten ist, so ist es im Rahmen des Herstellprozesses erfindungsgemäß bevorzugt, Wachs in einer Menge mindestens eines Esters der Formel (I) (*vide supra*) zu lösen und dann in dieser gelösten Form in das erfindungsgemäße kosmetische Mittel einzuarbeiten.

Erfindungsgemäß ganz besonders bevorzugt, enthält das erfindungsgemäße Mittel mindestens ein Wachs, ausgewählt aus mindestens einem Wachs der Gruppe Wachse mit der INCl-Bezeichnung Cocoglycerides, Cetylpalmitat, Myristylmyristat Butyrospermum Parkii Butter.

Die erfindungsgemäßen Mittel enthalten bevorzugt Wachs in einer Gesamtmenge von 0,01 bis 10,0 Gew.-%, besonders bevorzugt von 0,1 bis 5,0 Gew.-%, ganz besonders bevorzugt von 0,5 bis 3,0 Gew.-%, jeweils bezogen auf das Gesamtgewicht des Mittels.

Die erfindungsgemäßen Zusammensetzungen enthalten mindestens einen Emulgator in einer Gesamtmenge von 0,03 bis 10,0 Gew.-%, bevorzugt von 0,1 bis 10,0 Gew.%, mehr bevorzugt von 0,5 bis 7,5 Gew.%, noch bevorzugter von 0,5 bis 5,0 Gew.%, besonders bevorzugt von 1,0 bis 5,0 Gew.% und höchst bevorzugt von 1,0 bis 3,0 Gew.% und allerhöchst bevorzugt von 1,0 bis 1,5 Gew.%.

Der erfinungsgemäße Emulgator ist ausgewählt aus den Emulgatoren mit den INCI - Bezeichnungen "Glyceryl Oleate Citrate, Caprylic/Capric Triglyceride", "Polyglyceryl-4 Laurate/Succinate", "Polyglyceryl-4 Laurate/Sebacate (and) Polyglyceryl-4 Caprylate/Caprate", "Glyceryl Stearate (and) Polyglyceryl-6 Palmitate/ Succinate (and) Cetearyl Alcohol", Polyglyceryl-4 Laurate/Sebacate (and) Polyglyceryl-6 Caprylate/Caprate" sowie deren Mischungen.

Mit Vorzug wird der Emulgator ausgewählt aus "Glyceryl Oleate Citrate, Caprylic/Capric Triglyceride", "Polyglyceryl-4 Laurate/Succinate", "Polyglyceryl-4 Laurate/Sebacate (and) Polyglyceryl-4 Caprylate/Caprate" und "Polyglyceryl-4 Laurate/Sebacate (and) Polyglyceryl-6 Caprylate/Caprate" sowie deren Mischungen.

Besonders bevorzugt wird der Emulgator ausgewählt aus den Emulgatoren "Glyceryl Oleate Citrate, Caprylic/Capric Triglyceride", "Polyglyceryl-4 Laurate/Succinate" sowie deren Mischungen.

Höchst bevorzugt wird der Emulgator "Glyceryl Oleate Citrate, Caprylic/Capric Triglyceride" verwendet.

Die erfindungsgemäßen Mittel weisen eine Viskosität von 20.000 bis 300.000 mPas, insbesondere von 30.000 bis 200.000 mPas (jeweils gemessen bei 20°C, Brookfield, 4 Umdrehungen pro Minute, Spindel TE) auf.

In einer Ausführungsform können die kosmetischen Mittel beispielsweise als Antitranspirant, Lippenstift, UV-Schutzkosmetikum etc. verwendet werden. Die erfindungsgemäßen kosmetischen Mittel enthalten in einer dieser Ausführungsformen partikelförmig dispergierten Feststoff. Im Sinne der Erfindung sind Partikel als Korn vorliegende Teilchen (vgl. DIN 66160: 1992-09) von Festkörpern. In dem erfindungsgemäßen Mittel liegen folglich als Korn vorliegende Teilchen von Festkörpern als solche dispergiert vor.

Die Partikel weisen bevorzugt einen mittleren Teilchendurchmesser (Volumenmittel) von 0.01 bis 3,0 µm, insbesondere von 0,05 bis 1,0 µm, ganz besonders bevorzugt von 0,1 bis 0,5 µm.

Es sind erfindungsgemäß solche kosmetischen Mittel bevorzugt, die partikelförmig dispergierten Feststoff in einer Gesamtmenge von 10 bis 30 Gew.-% enthalten.

Als partikelförmig dispergierter Feststoff ist in bevorzugten erfindungsgemäßen kosmetischen Mitteln mindestens ein anorganischer Feststoff, insbesondere mindestens ein Metalloxid, enthalten. Die Metalloxide werden wiederum bevorzugt ausgewählt aus Aluminaten, Aluminiumsilikaten, Titandioxid, Zinkdioxid, Eisenoxiden, Zinndioxid, Perlglanzpigmenten sowie Gemischen aus zwei oder mehreren der vorgenannten Metalloxide.

Häufig verwendete Perlglanzpigmente sind Perlmutt (aus vermahlenen Muschelschalen), monokristalline Perlglanzpigmente wie z.B. Bismutoxychlorid, sowie Perlglanzpigmente auf Basis von Glimmer, Glimmer/Metalloxid oder Titandioxid/Metalloxid. Letztgenannte Perlglanzmischpigmente werden mit einer vom Partikelmaterial des Kerns verschiedenen Metalloxidbeschichtung versehen. Durch den Einsatz der Perlglanzpigmente werden Farbeffekte und/oder Glanz in den erfindungsgemäßen Mitteln oder auf dem damit behandelten Substrat erzielt.

Besonders bevorzugte Aluminate werden ausgewählt unter aktivem Aluminiumoxid, alpha-Aluminiumoxid, beta-Aluminiumoxid, gamma-Aluminiumoxid sowie deren Gemischen.

Besonders bevorzugte Aluminiumsilikate (auch Alumosilikate genannt) werden ausgewählt unter Phyllosilikaten, Tectosilikaten.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin).

Bevorzugte Titandioxide sind solche, die unter dem Handelsnamen Kronos von der Firma Kronos vertrieben werden, insbesondere Kronos 1171.

Bevorzugte Eisendioxide sind Fe₂O₃, beispielsweise mit der INCl Cl 77491, insbesondere als Unipur Red LC 281 EM^{®} von der Fa. Sensient im Handel, Fe₂O₃·nH₂O, beispielsweise mit der INCl Cl 77491, insbesondere als Unipur Red LC 281 EM^{®} von der Fa. Sensient im Handel, FeO·Fe₂O₃, beispielsweise mit der INCl Cl 77499, insbesondere als Unipur Black LC 989 EM^{®} von der Fa. Sensient im Handel.

Erfindungsgemäß bevorzugte kosmetische Mittel sind dadurch gekennzeichnet, dass sie (gemeinsam mit vorgenannten anorganischen Feststoffen oder ohne die Gegenwart von vorgenannten anorganischen Feststoffen als partikelförmig dispergierten Feststoff) als partikelförmig dispergierten Feststoff mindestens eine Stärke enthält.

Stärke ist ein Reservekohlenhydrat, das von vielen Pflanzen in Form von üblicherweise 1 bis 200 µm großen Stärkekörnern (Granula) in verschiedenen Pflanzenteilen gespeichert wird, z.B. in Knollen oder Wurzeln, Getreidesamen, Früchten sowie im Mark. Stärke gehört zu der Familie der Homoglycane und ist ein Polykondensationsprodukt von D-Glucose. Dabei besteht Stärke aus drei strukturell verschiedenen Polymeren der d-Glucopyranose, nämlich der Amylose, dem Amylopektin und einer sogenannten Zwischenfraktion. Höhere Pflanzen enthalten 0 bis 45 Gew.-% Amylose bezogen auf die Trockensubstanz. Die Zwischenfraktion, die auch als anormales Amylopektin bezeichnet wird, steht strukturell zwischen der Amylose und dem Amylopektin. Die im Rahmen dieser Anmeldung definierten Mengenangaben für Amylopektin umfassen die Zwischenfraktion. Amylose besteht aus überwiegend linear α-1,4-glycosidisch verknüpfter d-Glucose, Mr 50000-150000. Die resultierenden Ketten bilden in der Stärke Doppelhelices. Amylopektin enthält neben den für Amylose beschriebenen α-1,4-Verknüpfungen auch in einer Menge von 4 bis 6% α-1,6-Bindungen als Verzweigungsstellen. Der durchschnittliche Abstand zwischen den Verzweigungsstellen beträgt etwa 12 bis 17 Glucose-Einheiten. Die Molmasse von 107 bis 7·108 entspricht ca. 105 Glucose-Einheiten, womit Amylopektin zu den größten Biopolymeren gehört. Besagte Verzweigungen sind derart über das Molekül verteilt, dass sich eine Büschelstruktur mit relativ kurzen Seitenketten entwickelt. Zwei dieser Seitenketten bilden jeweils eine Doppelhelix. Durch die vielen Verzweigungsstellen ist Amylopektin in Wasser relativ gut löslich.

Eine erfindungsgemäß bevorzugt verwendbare Stärke wird ausgewählt unter mindestens einem Polykondensationsprodukt von D-Glucose erhalten aus Stärke von Kartoffeln, Mais, Reis, Erbsen, Eicheln, Kastanien, Gerste, Weizen, Bananen, Sago, Hirse, Sorghum, Hafer, Gerste, Roggen, Bohnen, Batate, Maranta oder Maniok. Besonders bevorzugt enthält das erfindungsgemäße Mittel mindestens eine Stärke, die Tapioka Stärke, Kartoffelstärke, Maisstärke oder Reisstärke ist. Gemische der vorgenannten Stärkeverbindungen sind erfindungsgemäß ebenso umfasst. Ganz besonders bevorzugt ist Tapiokastärke.

Die Stärkeverbindung ist in den erfindungsgemäßen Mitteln bevorzugt in einer Menge von 0,05 bis 8,0 Gew.-%, insbesondere von 0,5 bis 7,0 Gew.-%, jeweils bezogen auf das Gewicht des Mittels, enthalten.

Im Rahmen einer weiteren Ausführungsform der Erfindung ist es erfindungsgemäß bevorzugt, wenn der partikelförmig dispergierte Feststoff an der Oberfläche des Feststoffpartikels mit mindestens einem Wasser-in-Öl-Emulgator beschichtet ist.

Besonders geeignete Wasser-in-Öl-Emulgatoren werden bevorzugt ausgewählt aus mindestens einer Verbindung aus der Gruppe, die gebildet wird aus
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈-C₃₀-Fettsäureresten verestert,
- lineare oder verzweigte, gesättigte oder ungesättigte C₁₂-C₃₀-Alkanole, die jeweils mit 1-4 Ethylenoxid-Einheiten pro Molekül verethert sind, welche ausserordentlich bevorzugt sind aus Steareth, Ceteth, Myristeth, Laureth, Trideceth, Arachideth und Beheneth mit jeweils 1-4 Ethylenoxid-Einheiten pro Molekül, insbesondere Steareth-2, Steareth-3, Steareth-4, Ceteth-2, Ceteth-3, Ceteth-4, Myristeth-2, Myristeth-3, Myristeth-4, Laureth-2, Laureth-3, Laureth-4, Trideceth-2, Trideceth-3 und Trideceth-4 sowie Mischungen hiervon;
- lineare gesättigte Alkanole mit 12-30 Kohlenstoffatomen, insbesondere mit 16-22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind;
- Ester und insbesondere Partialester aus einem Polyol mit 2-6 C-Atomen und linearen gesättigten und ungesättigten Fettsäuren mit 12-30, insbesondere 14-22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂-C₃₀-Carbonsäuren, die hydroxyliert sein können, insbesondere diejenigen mit Palmitin- und Stearinsäure, die Sorbitanmono-, - di- oder -triester von linearen gesättigten und ungesättigten C₁₂-C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, - tri- und -tetraester und die Methylglucosemono- und -diester von linearen, gesättigten und ungesättigten C₁₂-C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12-30, insbesondere 14-22 Kohlenstoffatomen, die hydroxyliert sein können, sowie Mischungen hiervon, als Konsistenzgeber und/oder Wasserbinder. Erfindungsgemäss besonders bevorzugt sind die Mono- und Diester. Erfindungsgemäss bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1-5 EO) sein können;
- Alkanole und Carbonsäuren mit jeweils 8-24 C-Atomen, insbesondere mit 16-22 C-Atomen, in der Alkylgruppe und 1-4 Ethylenoxid-Einheiten pro Molekül, die einen HLB-Wert grösser 1,0 und kleiner/gleich 7,0 aufweisen,
- Glycerinmonoether gesättigter und/oder ungesättigter, verzweigter und/oder unverzweigter Alkohole einer Kettenlänge von 8-30, insbesondere 12-18 Kohlenstoffatomen.

Flüssige Partialester von Polyglycerinen mit n = 2 bis 10 Glycerineinheiten und mit 1 bis 5 gesättigten oder ungesättigten, linearen oder verzweigten, gegebenenfalls hydroxylierten C₈-C₃₀-Fettsäureresten verestert, sind erfindungsgemäß besonders bevorzugte Wasser-in-Öl-Emulgatoren zur Auswahl. Ganz besonders bevorzugt wird der Wasser-in-Öl Emulgator ausgewählt aus mindestens einem Emulgator, der 2 bis 6 aneinander kovalent bindende Glyceryleinheiten und mindestens eine (insbesondere verzweigte) Alkylgruppe mit 8 bis 20 Kohlenstoffatomen umfasst.

In einer bevorzugten Ausführungsform der vorliegenden Erfindung sind in den erfindungsgemäßen Mitteln partikelförmige Teilchen enthalten. In dieser bevorzugten Ausführungsform ist die Einstellung der Viskosität wesentlich. Die erfindungsgemäßen Mittel dieser Ausführungsform weisen, wenn sie partikelförmige Teilchen enthalten, eine Viskosität von 300000 bis 800000 mPas, insbesondere von 400000 bis 600000 mPas (jeweils gemessen bei 20°C, Brookfield, 4 Umdrehungen pro Minute, Spindel TE) auf.

Diese Viskositäten können sich auch ohne Zusatz eines zusätzlichen Verdickungsmittels für die Verdickung von Öl einstellen. Sollte dies nicht der Fall sein, empfiehlt es sich, der Rezeptur ein Verdickungsmittel für die Verdickung von Öl zuzusetzen. Weitere bevorzugte erfindungsgemäße kosmetische Mittel sind daher dadurch gekennzeichnet, dass sie zusätzlich mindestens ein Verdickungsmittel für die Verdickung von Öl enthalten. Diese zusätzlichen Verdickungsmittel für die Verdickung von Öl sind von den vorgenannten zwingend in dem erfindungsgemäßen Mittel enthaltenen Komponenten verschieden.

Es ist erfindungsgemäß zweckmäßig, dass, falls die zusätzlichen Verdickungsmittel für die Verdickung von Öl in dem erfindungsgemäßen kosmetischen Mittel Anwendung finden, besagte Verdickungsmittel nur in einer solchen Menge zugesetzt werden, bis die Viskosität im erfindungsgemäßen Viskositätsbereich liegt.

Erfindungsgemäß bevorzugte Verdickungsmittel für die Verdickung von Öl sind ausgewählt aus hydrophobierten Tonmineralien, Kieselsäuren (insbesondere pyrogenen Kieselsäuren), Ethylene/Propylene/Styrene Copolymeren, Butylene/ Ethylene/Styrene Copolymeren, Dextrinestern und/oder Siliconelastomeren.

Bevorzugte hydrophobierte Tonmineralien sind ausgewählt aus hydrophobierten Montmorilloniten, hydrophobierten Hectoriten und hydrophobierten Bentoniten, besonders bevorzugt aus Disteardimonium Hectorite, Stearalkonium Hectorite, Quaternium-18 Hectorite und Quaternium-18 Bentonite. Die handelsüblichen Verdickungsmittel stellen diese hydrophobierten Tonmineralien in Form eines Gels in einer Ölkomponente, bevorzugt in Cyclomethicone und/oder einer Nichtsilicon-Ölkomponente, wie z. B. Propylencarbonat, bereit. Derartige Gele sind beispielsweise unter der Handelsbezeichnung Bentone^{®} oder Thixogel erhältlich.

Erfindungsgemäß bevorzugte Zusammensetzungen sind dadurch gekennzeichnet, dass sie hydrophobiertes Tonmineral in einer Gesamtmenge von 0,5 - 10 Gew.-%, bevorzugt 1 - 7 Gew.-%, besonders bevorzugt 2 - 6 Gew.-%, außerordentlich bevorzugt 3 - 5 Gew.-%, jeweils bezogen auf das Gesamtgewicht der erfindungsgemäßen Zusammensetzung, enthalten.

Weitere erfindungsgemäß ganz besonders bevorzugte Verdickungsmittel für die Verdickung von Öl sind ausgewählt aus Kieselsäure, insbesondere pyrogene Kieselsäure (z. B. den Handelsprodukten der Aerosil^{®}-Serie von Evonik Degussa). Diese können an deren Oberfläche durch chemische Modifikation hydrophobiert sein (wie z.B. die silylierten Kieselsäuren mit der INCI-Bezeichnung Silica Silylate) was jedoch nicht bevorzugt ist. Erfindungsgemäß besonders bevorzugt geeignete pyrogene Kieselsäure hat die INCl-Bezeichung Silica.

Wird zusätzlich pyrogener Kieselsäure als Verdickungsmittel für die Verdickung von Öl eingesetzt, werden besonders lagerstabile kosmetische Mittel der vorliegenden Erfindung erhalten.

Erfindungsgemäß bevorzugte kosmetische Mittel sind dadurch gekennzeichnet, dass sie Kieselsäure, bevorzugt pyrogene Kieselsäure, in einer Gesamtmenge von 0,1 bis 6 Gew.-%, bevorzugt 0,5 bis 5 Gew.-%, besonders bevorzugt 1,0 bis 4,5 Gew.-%, ganz besonders bevorzugt 1,5 bis 3,0 Gew.-%, außerordentlich bevorzugt 1,9 bis 2,6 Gew.-%, jeweils bezogen auf das Gesamtgewicht der erfindungsgemäßen Zusammensetzung, enthalten.

Weitere erfindungsgemäß einsetzbare lipophile Verdickungsmittel sind ausgewählt aus Ethylene/ Propylene/Styrene Copolymeren und Butylene/Ethylene/Styrene Copolymeren. Besonders bevorugt werden die Copolymere als vorverdicktes öl-basiertes Gel eingesetzt. Derartige Gele sind beispielsweise unter der Handelsbezeichnung Versagel^{®} (ex Penreco) erhältlich. Bevorzugt sind Gele mit Mineralöl, hydriertem Polyisobuten, Isoparaffinen, wie Isohexadecan oder Isododecan, sowie mit Esterölen, insbesondere mit Isopropylpalmitat oder Isopropylmyristat.

Weitere erfindungsgemäß einsetzbare lipophile Verdickungsmittel sind ausgewählt aus Siliconelastomeren. Eine weitere bevorzugte Ausführungsform der Erfindung ist dadurch gekennzeichnet, dass mindestens ein Siliconelastomer, das erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens 2 Silicon-gebundene Wasserstoffatome in jedem Molekül aufweist, enthalten ist.

Erfindungsgemäß besonders bevorzugte Organopolysiloxane mit mindestens 2 C₂ - C₁₀ - AlkenylGruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethyl-siloxy-Endgruppen und cyclischen Dimethylsiloxan-Methylhydrogen-siloxan-Copolymeren.

Erfindungsgemäß besonders bevorzugte Siliconelastomere, die als Rohstoff bereits in einem bei Raumtemperatur unter Normalbedingungen flüssigen Silicon vorgequollen vorliegen und ein Silicon-basiertes Gel darstellen, sind kommerziell erhältlich, beispielsweise unter den Handelsnamen Dow Corning 9040 Silicone Elastomer Blend (ein Cyclomethicone (and) Dimethicone Crosspolymer von Dow Corning; Silikonelastomergehalt 12 - 13 Gew.-%), SFE 168, ein Cyclomethicone (and) Dimethicone/Vinyl Dimethicone Crosspolymer von GE Silicones, Vinyl Dimethicone Crosspolymere, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/Phenyl Vinyl Dimethicone Crosspolymer, Siliconelastomergehalt 10 - 20 Gew.-%); and KSG-20, erhältlich von Shin Etsu Silicones of America (Akron, Ohio), und von Grant Industries Inc. (Elmwood Park, NJ) die Produkte aus der Gransil^{®}-Serie, insbesondere Gransil SR-CYC (Cyclomethicone and Stearyl-Vinyl/hydromethylsiloxane Copolymer), Gransil^{®} RPS Gel (INCl-Bezeichnung: Cyclopentasiloxane and Polysilicone-11), Gransil^{®} GCM-4 (INCI-Bezeichnung: Cyclotetrasiloxane and Polysilicone-11), Gransil^{®}GCM-5 (INCl-Bezeichnung: Cyclopentasiloxane and Polysilicone-11), Gransil^{®} RPS (INCl-Bezeichnung: Cyclopentasiloxane and Polysilicone-11), GI-CD 10 (INCl-Bezeichnung: Cyclopentasiloxane (and) Stearoxymethicone/Dimethicone Copolymer (and) Dimethicone), Gransil^{®} IDS (INCl-Bezeichnung: Isododecane (and) Cyclotetrasiloxane (and) Polysilicone-11), Gransil^{®}PC-12 (INCl-Bezeichnung: Isododecane (and) Polysilicone-11), Gransil^{®}IDS-5 (INCl-Bezeichnung: Isododecane (and) Cyclopentasiloxane (and) Polysilicone-11), Gransil^{®}APK-1 (INCl-Bezeichnung: Dimethicone and Cyclopentasiloxane and Polysilicone-11 and Nylon-12 and Methyl Methacrylate/Acrylonitrile Copolymer and PEG-10 Dimethicone and Polysorbate-40 and Isohexadecane and Ammonium Polyacryloyldimethyl Taurate), Gransil^{®}DMCM-5 (INCl-Bezeichnung: Dimethicone and Cyclopentasiloxane and Polysilicone-11), Gransil^{®}DMG-6 mit Dimethicone (6 cSt) (INCl-Bezeichnung: Dimethicone and Polysilicone-11), Gransil^{®}DMG-20 mit Dimethicone (20 cSt) (INCl-Bezeichnung: Dimethicone and Polysilicone-11), Gransil^{®} AM-8 Gel (INCl-Bezeichnung: Caprylyl Methicone and Cyclopentasiloxane and Polysilicone-11), Gransil^{®} DM 5 mit Dimethicone (5 cSt) (INCI-Bezeichnung: Dimethicone and Polysilicone-11), Gransil^{®} DMID (INCl-Bezeichnung: Dimethicone and Isododecane and Polysilicone-11), Gransil^{®} PM (INCl-Bezeichnung: Phenyl Trimethicone and Polysilicone-11), Gransil^{®} ININ (INCl-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Siliconöl, Fett oder Wachs, vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil^{®} MLB (INCl-Bezeichnung: Cyclopentasiloxane and Polysilicone-11 and Beeswax), Gransil^{®} PS (INCl-Bezeichnung: Cyclotetrasiloxane and Polysilicone-11 and Petrolatum), Gransil^{®} PS-5 (INCl-Bezeichnung: Cyclopentasiloxane and Polysilicone-11 and Petrolatum), Gransil^{®} DMG-20 P mit Dimethicone (20 cSt) und Petrolatum (INCl-Bezeichnung: Dimethicone and Polysilicone-11 and Petrolatum), Gransil^{®} RJO (INCl-Bezeichnung: Cyclopentasiloxane and Polysilicone-11 and Jojoba Oil), Gransil^{®} LANO (INCl-Bezeichnung: Cyclopentasiloxane and Polysilicone-11 and Lanolin), Gransil^{®} OHS-5 (INCI-Bezeichnung: Cyclopentasiloxane and Polysilicone-11 and Octyl Hydroxystearate) und Gransil^{®} DML (INCl-Bezeichnung: Dimethicone (and) Neopentyl Glycol Diheptanoate (and) Polysilicone-11).

Eine weitere bevorzugte Ausführungsform der Erfindung ist dadurch gekennzeichnet, dass das Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit x = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien.

Erfindungsgemäß bevorzugte Zusammensetzungen sind dadurch gekennzeichnet, dass sie mindestens ein Siliconelastomer in einer Gesamtmenge von 0,05 - 3 Gew.-%, bevorzugt 0,1 - 2 Gew.-%, besonders bevorzugt 0,2 - 1,0 Gew.-%, außerordentlich bevorzugt 0,3 - 0,5 Gew.-%, jeweils bezogen auf das Gesamtgewicht der erfindungsgemäßen Zusammensetzung, enthalten.

### Beispiele

Es wurden folgende kosmetische Mittel gemäß Erfindung hergestellt:

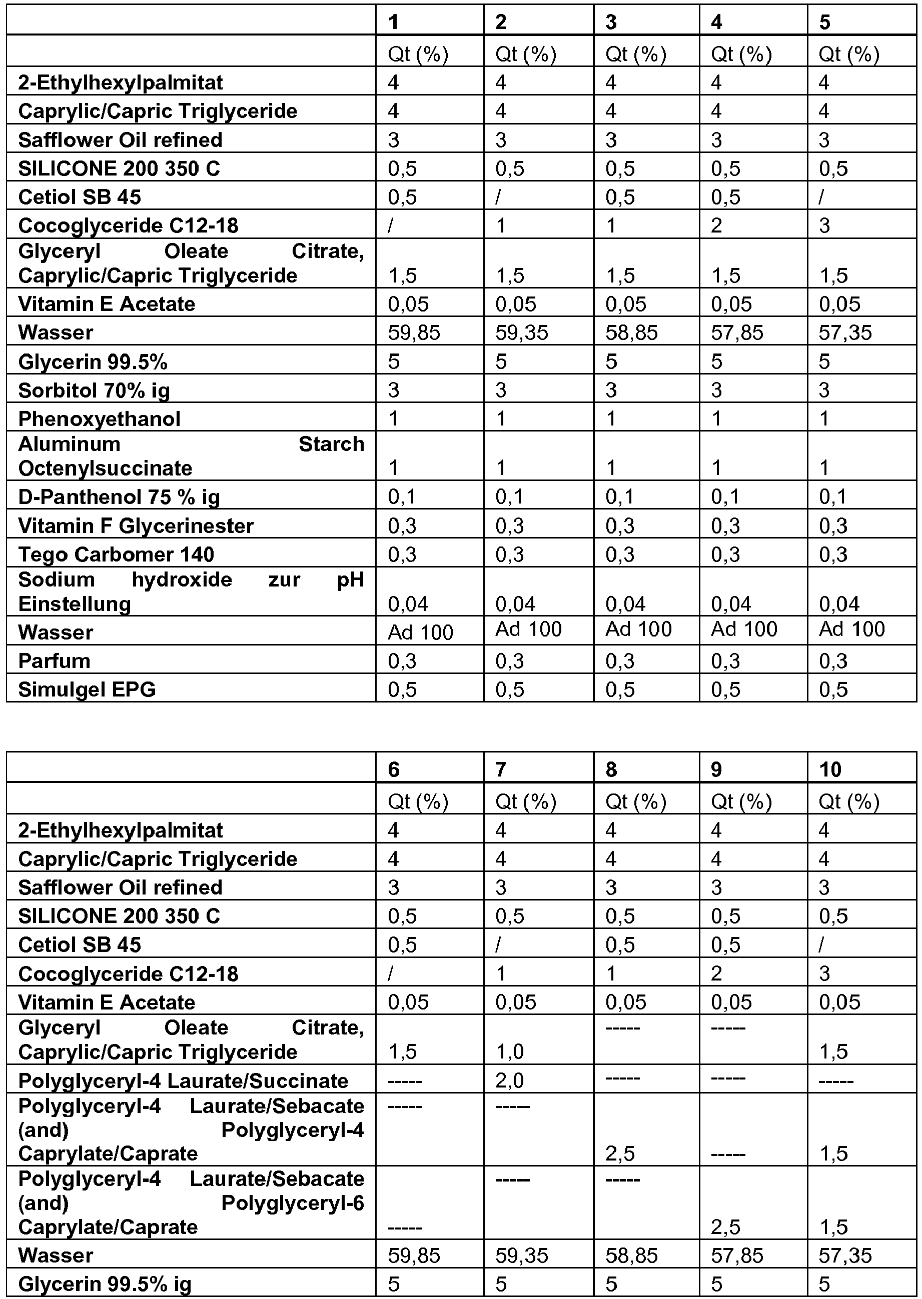

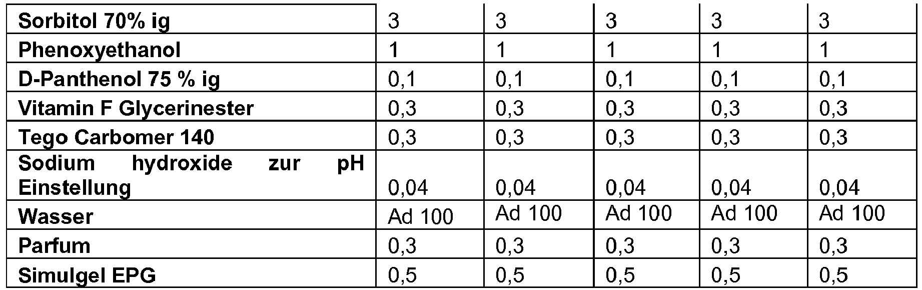

In einem Rührgefäß (Symex 1000 der Firma Schröder & Boos GmbH & Co. KG, Deutschland) mit Rührer und Homogenisator wurde bei einer Temperatur von 25°C Wachs in der flüssigen Ölkomponente unter Rühren gelöst.

Es wurden unter Rühren die Rohstoffe der Nummer 2 gemäß Tabelle 2 zugegeben.

Bei einer Temperatur von 25°C wurden die Inhaltsstoffe der Nummer 4 gemäß Tabelle 1 unter Rühren zugegeben.

## Patentansprüche

1. Kosmetische Mittel, enthaltend in einem geeigneten kosmetischen Träger jeweils bezogen auf das Gesamtgewicht des Mittels
- ein oder mehrere flüssige Öle in einer Gesamtmenge von 0,1 bis 30,0 Gew.-%,
- ein oder mehrere Wachse mit einem Schmelzpunkt von 20 bis 90 °C in einer Gesamtmenge von 0,01 bis 10,0 Gew.-%,
- mindestens einen Emulgator, ausgewählt aus den Emulgatoren mit den INCI - Bezeichnungen "Glyceryl Oleate Citrate, Caprylic/Capric Triglyceride", "Polyglyceryl-4 Laurate/Succinate", "Polyglyceryl-4 Laurate/Sebacate (and) Polyglyceryl-4 Caprylate/Caprate", "Glyceryl Stearate (and) Polyglyceryl-6 Palmitate/ Succinate (and) Cetearyl Alcohol Polyglyceryl-4 Laurate/Sebacate (and) Polyglyceryl-6 Caprylate/Caprate" sowie deren Mischungen in einer Gesamtmenge von 0,03 bis 10,0 Gew.-% und
- gegebenenfalls weitere Inhaltsstoffe,
- und **dadurch gekennzeichnet, dass** die Herstellung der Mittel ausschließlich in einem Kaltverfahren erfolgt.

2. Kosmetisches Mittel nach Anspruch 1, **dadurch gekennzeichnet**, das flüssige Öl ausgewählt aus mindestens einer Verbindung der Gruppe, die gebildet wird aus flüssigen Silikonen; Esterölen; Trifettsäureester von gesättigten und/oder ungesättigten, linearen und/oder verzweigten C₆- bis C₂₂-Fettsäuren mit Glycerin; pflanzlichen Ölen; flüssigen Paraffinölen; Isoparaffinölen; flüssigen synthetischen Kohlenwasserstoffen; flüssigen Di-n-alkylethern; Dicarbonsäureestern; symmetrischen, unsymmetrischen oder cyclischen Estern der Kohlensäure.

3. Kosmetisches Mittel nach einem der vorangehenden Ansprüche, **dadurch gekennzeichnet, dass** es als flüssiges Öl mindestens einen Ester der Formel (I) enthält 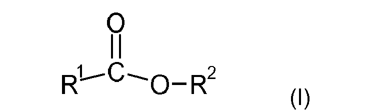 worin R¹ und R² unabhängig voneinander für einen linearen oder verzweigten Kohlenwasserstoffrest mit 3 bis 22 Kohlenstoffatomen stehen.

4. Kosmetisches Mittel, nach einem der vorangehenden Ansprüche, **dadurch gekennzeichnet, dass** es Wachs in einer Gesamtmenge von 0,01 bis 10,0 Gew.-%, bevorzugt von 0,1 bis 5,0 Gew.-%, besonders bevorzugt von 0,5 bis 3,0 Gew.-%, enthält.

5. Kosmetisches Mittel nach einem der vorangehenden Ansprüche, **dadurch gekennzeichnet, dass** es als Wachs mindestens ein Wachs mit einem Schmelzpunkt von 30 bis 50°C, insbesondere von 30 bis 40°C, enthält.

6. Kosmetisches Mittel, nach einem der vorangehenden Ansprüche, **dadurch gekennzeichnet, dass** das Wachs, ausgewählt ist aus mindestens einem Wachs der Gruppe Wachse mit der INCl-Bezeichnung Cocoglycerides, Cetylpalmitat, Myristylmyristat Butyrospermum Parkii Butter.

7. Kosmetisches Mittel nach einem der vorangehenden Ansprüche, **dadurch gekennzeichnet, dass** mindestens die Hälfte der Menge des enthaltenen Wachses (bevorzugt mindestens 75% des enthaltenen Wachses) gelöst vorliegt.

8. Kosmetisches Mittel nach einem der vorangehenden Ansprüche, **dadurch gekennzeichnet, dass** der Emulgator ausgewählt ist aus Glyceryl Oleate Citrate, Caprylic/Capric Triglyceride" und "Polyglyceryl-4 Laurate/Succinate" sowie deren Mischungen.

9. Kosmetisches Mittel nach einem der vorangehenden Ansprüche, **dadurch gekennzeichnet, dass** der Emulgator "Glyceryl Oleate Citrate, Caprylic/Capric Triglyceride" enthalten ist.

10. Kosmetisches Mittel nach einem der vorangehenden Ansprüche, **dadurch gekennzeichnet, dass** es partikelförmig dispergierten Feststoff in einer Gesamtmenge von 10 bis 30 Gew.-% enthält.
